(19) Europäisches Patentamt
European Patent Office
Office européen des brevets

(11) Veröffentlichungsnummer: **0 140 254 B1**

(12) **EUROPÄISCHE PATENTSCHRIFT**

(45) Veröffentlichungstag der Patentschrift: **05.10.88**

(51) Int. Cl.[4]: **C 07 B 37/02** // C07C19/08, C07C69/63

(21) Anmeldenummer: **84112300.3**

(22) Anmeldetag: **12.10.84**

(54) **Verfahren zur Herstellung von fluoralkylsubstituierten Iod-Alkanen.**

(30) Priorität: **21.10.83 DE 3338300**

(43) Veröffentlichungstag der Anmeldung: **08.05.85 Patentblatt 85/19**

(45) Bekanntmachung des Hinweises auf die Patenterteilung: **05.10.88 Patentblatt 88/40**

(84) Benannte Vertragsstaaten: **BE CH DE FR GB IT LI NL**

(56) Entgegenhaltungen: **EP - A - 0 090 712**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **von Werner, Konrad, Dr., Buch 1 1/2, D-8261 Halsbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von fluoralkylsubstituierten Iod-Alkanen gemäss Anspruch 1.

Es ist bekannt, Iodperfluoralkane an die C–C-Doppelbindung von Alkenen, die verschiedene Substituenten tragen können, anzulagern. Hierbei wurden verschiedenen Wege beschritten. Die rein thermische Anlagerung benötigt hohe Temperaturen, führt oft nur zu mässigen Umsätzen und verläuft im allgemeinen wenig selektiv, das heisst, es entsteht ein Gemisch verschiedener Anlagerungsprodukte.

Bekannt ist auch die photochemische Anlagerung von Perfluoralkyliodiden an Alkene, die allerdings oft lange Reaktionszeiten erfordert, um zu brauchbaren Umsätzen zu kommen, ausserdem technisch umständlich und energieaufwendig ist.

Weiterhin ist bekannt, Perfluoralkyliodide unter Verwendung von bekannten, radikalisch zerfallenden Katalysatoren wie Azobis-isobutyronitril oder organischen Peroxiden in aprotischen Lösungsmitteln an Alkene anzulagern. Die Reaktionstemperaturen sind gegenüber der thermischen Anlagerung erheblich herabgesetzt, es werden auch häufig gute Umsätze und Ausbeuten von den Reaktionsprodukten erhalten. Das Verfahren hat jedoch den Nachteil, dass Reste des Katalysators und insbesondere seiner Zersetzungsprodukte in der Reaktionsmischung verbleiben und damit die Abtrennung und Reindarstellung der gewünschten Reaktionsprodukte erschweren. Die Anlagerungsreaktion verläuft zudem häufig nicht besonders selektiv, was die Ausbeute an erwünschten Produkten schmälert.

Schliesslich ist es bekannt, Perfluoralkylkupfer(I) in aprotischem Lösungsmittel (Dimethylsulfoxid) mit Alkenen umzusetzen, wobei ebenfalls vergleichsweise niedrige Reaktionstemperaturen ausreichend sind, allerdings wiederum Gemische von verschiedenen Reaktionsprodukten entstehen, die die Reindarstellung der einzelnen Produkte erschweren und die Ausbeute an erwünschten Produkten mindern. Das Perfluoralkylkupfer(I) kann auch als Perfluoralkyliodid und metallischem Kupfer, wiederum unter Verwendung eines aprotischen Lösungsmittels (Dimethylsulfoxid), erst in der Reaktionsmischung erzeugt werden, wobei auch weniger (beispielsweise nur ein Drittel) der stöchiometrisch berechneten Menge des metallischen Kupfers ausreichen. Jedoch werden auch bei dieser Reaktionsart in nicht unbeträchtlicher Menge Nebenprodukte gebildet, wie nachfolgende Vergleichsversuche zeigen.

In der EP 90 712-A2 wird ein Verfahren zur Herstellung von verzweigten Bis-(perfluoralkyl)-1,2-ethenen beschrieben, wobei nach den Beispielen zunächst ein Perfluoralkylethen mit einem Perfluoralkyliodid ohne Verwendung eines Katalysators bei Temperaturen von 190 °C und darüber in einem Autoklaven während längerer Zeit behandelt und das erzeugte fluoralkyl-substituierte Iod-Alkan anschliessend mit Alkalihydroxid zum Bis-(perfluoralkyl)-1,2-ethen umgesetzt wird.

Nach der Beschreibung kann ein Perfluoralkyliodid mit einem Brom-1-perfluoralkyl-1-ethen in Gegenwart eines Lösungsmittels, wie Dimethylformamid, in Gegenwart von Kupfer direkt zum Bis-(perfluoralkyl)-1,2-ethen umgesetzt werden. Nähere Angaben über die Reaktionsbedingungen fehlen. Wie eingangs bereits erwähnt, hat die thermische Anlagerung bei hohen Temperaturen während beträchtlicher Zeiten Nachteile, da sie die Bildung unerwünschter Nebenprodukte begünstigt. Die Umsetzung mit Kupfer führt nach der Lehre der EP 90 712-A2 nicht zum fluoralkylsubstituierten Iod-Alkan, sondern zum Bis-(perfluoralkyl)-1,2-ethen.

Es wurde nun ein Verfahren gefunden, welches ermöglicht, ein ganz oder überwiegend mit Fluor substituiertes Alkyliodid an Alkene so anzulagern, dass in guten Ausbeuten im wesentlichen nur ein Anlagerungsprodukt entsteht, welches leicht gereinigt und zu technisch interessanten Produkten weiter umgesetzt werden kann. Ein anderer Vorteil ist die problemlose Abtrennbarkeit und Wiederverwendbarkeit des eingesetzten Katalysators.

Es handelt sich um ein Verfahren zur Herstellung von fluoralkyl-substituierten Iod-Alkanen durch Reaktion eines Verfahrens zur Herstellung von fluoralkyl-substituierten Iod-Alkanen durch Reaktion einer Verbindung der Formel

$$XR_fI \qquad (I)$$

worin bedeuten:

$R_f$ einen perfluorierten Alkylenrest, geradkettig mit 1 bis 15 C-Atomen, verzweigt mit 3 bis 15 C-Atomen oder cyclisch mit 4 bis 8 C-Atomen

X H; F; Cl; Br oder I,

mit einer Verbindung der Formel

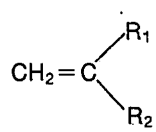

(II),

worin $R_1$ und $R_2$ gleich oder verschieden sein können und jedes für sich bedeutet:

Wasserstoff, Fluor, Chlor, einen Perfluoralkylrest mit 1 bis 12 C-Atomen, in dem ein Fluoratom durch Wasserstoff oder Chlor ersetzt sein kann; einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann, mit F; Cl; –OH oder –OR′, worin R′ einen Alkyl- oder Alkylcarboxyrest mit 1 bis 5 C-Atomen bedeutet, in der Wärme, bei Normaldruck oder Überdruck, in Gegenwart eines Katalysators, das dadurch gekennzeichnet ist, dass die Reaktion bei 80 bis 180 °C durchgeführt wird, wobei als Katalysator mindestens ein Metall, das im Periodensystem der Elemente eine der Ordungszahlen 24 bis 30; 42 bis 48 oder 74 bis 79 aufweist, in fein verteilter Form eingesetzt wird.

Unter «fluoralkylsubstituiertes Iod-Alkan» im Sinne der Erfindung ist eine solche Verbindung zu verstehen, die sich von einem aliphatischen Kohlenwasserstoff ableitet und an dem fluoralkylsubstituierten Kohlenstoffatom keine Doppelbindung aufweist. An anderer Stelle in der Kohlenstoffkette können Doppelbindungen auftreten, wenn zum Beispiel als Ausgangsprodukt für die Anlagerung ein Alken mit zwei Doppelbindungen im Molekül (Alkadien) eingesetzt wurde, jedoch die Anlagerung des fluorierten Alkyliodids nur an eine dieser Doppelbindungen erfolgt.

Als Katalysatoren geeignete Metalle sind beispielsweise Kobalt, Zink, Molybdän, Silber, Cadmium, Rhenium und Osmium. Besonders gute Ergebnisse werden mit Chrom, Mangan, Nickel, Ruthenium, Rhodium, Palladium oder Platin erhalten. Es können auch Gemische verschiedener Metalle (Legierungen) verwendet werden.

Die Metalle werden in fein verteilter Form, beispielsweise als Pulver oder Späne verwendet. Insbesondere bei teureren Metallen wie Ruthenium, Rhodium, Palladium oder Platin ist es vorteilhaft, diese auf einem feinteiligen, inerten Trägermaterial niedergeschlagen zu verwenden. Als Trägermaterial ist beispielsweise geeignet Aktivkohle, Aluminiumoxid oder Siliciumdioxid. Auch sogenannte Metallschwämme sind gut verwendbar.

Die Katalysatorteilchen können im Reaktionsgemisch gleichmässig verteilt verwendet werden oder in Form einer Packung (Festbett), welche von der Reaktionsmischung durchströmt wird.

Die Reaktion wird im Temperaturbereich von 80 bis 180 °C durchgeführt. Unterhalb 80 °C werden im allgemeinen zu geringe Ausbeuten erzielt, oberhalb 180 °C kann die Ausbeute an dem erwünschten Reaktionsprodukt im allgemeinen nicht mehr verbessert werden, im Gegenteil nimmt Zahl und Menge der gebildeten Nebenprodukte zu, so dass die Reindarstellung des erwünschten Produktes schwieriger und der Prozess unwirtschaftlich wird. Zweckmässig wird die Temperatur der Reaktionsmischung mindestens so hoch gehalten, dass die Reaktionspartner (mit Ausnahme des Katalysators) und die daraus entstehenden Produkte nicht in fester Form vorliegen. Vorzugsweise wird im Temperaturbereich von 100 bis 150 °C gearbeitet.

Das erfindungsgemässe Verfahren wird unter Druck ausgeführt, wenn mindestens einer der Reaktionspartner bei der gewählten Umsetzungstemperatur gasförmig ist. Zweckmässig wird unter dem autogenen Druck des oder der Reaktionspartner gearbeitet, es kann jedoch auch ein höherer Druck angewendet werden. Der Druckbereich, in dem das neue Verfahren ausgeführt wird, liegt zwischen 0,098 und 5 MPa. Nach oben ist der Druckbereich im wesentlichen nur durch wirtschaftliche Erwägungen begrenzt. Vorzugsweise wird im Druckbereich von 0,098 bis 2,5 MPa gearbeitet.

Die Dauer der Reaktion ist von der gewählten Temperatur und den Reaktionspartnern abhängig. Eine Reaktionsdauer 1 bis 50 h ist in der Regel ausreichend.

Unterhalb 1 h ist die Reaktion noch unvollständig, oberhalb 50 h wird im allgemeinen keine Ausbeuteverbesserung mehr beobachtet, es wächst die Gefahr der Bildung unerwünschter Nebenprodukte. Vorzugsweise wird eine Reaktionsdauer von 3 bis 30 h gewählt.

Das Molverhältnis der Reaktionspartner (fluoriertes Alkyliodid zu Alken) beträgt 10 : 1 bis 1 : 10. Im allgemeinen wird man eher einen molaren Überschuss des Alkens wählen, doch wächst dadurch die Gefahr, dass mehrere Alkenmoleküle mit einem fluorsubstituierten Alkyliodid reagieren, was zu weniger erwünschten Produkten führt. Es ist deshalb in einigen Fällen erforderlich, einen bisweiligen beträchtlichen molaren Überschuss des fluorierten Alkyliodids zu wählen. Vorzugsweise werden die Reaktionspartner im Molverhältnis 5 : 1 bis 1 : 5 eingesetzt.

Vom Katalysatormetall werden 0,1 bis 10 Mol-%, bezogen auf jene der beiden reagierenden Verbindungen, die mit geringerer Molzahl eingesetzt wird, angewendet. Unterhalb 0,1 Mol-% ist der Katalysator in der Regel nicht mehr ausreichend wirksam, oberhalb 10 Mol-% wird keine zusätzliche Wirkung des Katalysators mehr festgestellt. Vorzugsweise werden 1 bis 5 Mol-%, bezogen auf jene der beiden reagierenden Verbindungen, die mit geringerer Molzahl eingesetzt wird, vom Katalysator verwendet.

Das erfindungsgemässe Verfahren kann auch in Gegenwart von Lösungsmitteln durchgeführt werden, jedoch wird vorteilhaft in Abwesenheit von aprotischen Lösungsmitteln gearbeitet, da diese häufig die Bildung unerwünschter Nebenprodukte begünstigen. Protische Lösungsmittel stören überraschenderweise den Reaktionsablauf nur wenig oder gar nicht. Es wurde sogar festgestellt, dass sich ein Zusatz von Wasser (welches allerdings für die meisten Reaktionspartner kein Lösungsmittel ist) auf den Reaktionsablauf günstig auswirkt. Vorzugsweise werden der Reaktionsmischung 1 bis 100 Mol-%, bezogen auf jene der beiden reagierenden Verbindungen, die mit geringerer Molzahl eingesetzt wird, Wasser der Reaktionsmischung zugesetzt. Unter 1 Mol-% wird im allgemeinen keine Wirkung festgestellt, über 100 Mol-% tritt kein zusätzlicher Effekt mehr auf. Insbesondere werden 3 bis 30 Mol-%, bezogen auf jene der beiden reagierenden Verbindungen, die mit geringerer Molzahl eingesetzt wird, zugegeben.

Als fluoriertes Alkyliodid wird eine Verbindung folgender Formel eingesetzt:

$$XR_fI,$$

worin bedeuten

$R_f$ einen perfluorierten Alkylenrest, geradkettig mit 1 bis 15 C-Atomen oder verzweigt mit 3 bis 15 C-Atomen oder cyclisch mit 4 bis 8 C-Atomen;

X Wasserstoff, Fluor, Chlor, Brom oder Iod.

Die Verbindungen, in denen X Fluor oder Iod ist, werden bevorzugt eingesetzt, da sie gut reagieren und zu technisch interessanten Reaktionsprodukten führen. Aus den gleichen Gründen und wegen der leichten Zugänglichkeit werden Verbindungen bevorzugt, in denen $R_f$ einen perfluorierten geradkettigen Alkylenrest mit 2 bis 12 C-Atomen bedeutet.

Das erfindungsgemässe Verfahren wird ferner durchgeführt mit einem substituierten oder unsubstituierten Alken, das folgender Formel entspricht:

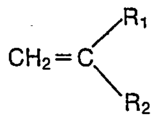

worin $R_1$ und $R_2$ gleich oder verschieden sein können und jedes für sich bedeutet:
Wasserstoff, Fluor, Chlor, einen Perfluoralkylrest mit 1 bis 12 C-Atomen, in dem ein Fluoratom durch Wasserstoff oder Chlor ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen. Ein Substituent der vier zuletzt genannten Arten (Alkyl-, Alkenyl-, Aryl- oder Arylalkyl-) kann seinerseits substituiert sein mit Fluor, Chlor, –OH oder –OR′, worin R′ einen Alkyl- oder Alkylcarboxy-Rest mit 1 bis 5 C-Atomen bedeutet. Ferner können $R_1$ oder $R_2$ einen Silyl-Rest bedeuten, der mit Alkylgruppen, die 1 bis 4 C-Atome enthalten, mit Alkoxygruppen, die 1 bis 5 C-Atome enthalten oder mit Chlor substituiert ist.

Besonders bevorzugt werden solche Verbindungen eingesetzt, in denen $R_2$ Wasserstoff bedeutet und $R_1$ ebenfalls Wasserstoff oder einen Alkylrest mit 1 bis 10 C-Atomen oder einen Alkenylrest mit 2 bis 10 C-Atomen, wobei sowohl der Alkyl- wie auch der Alkenylrest mit –OH oder –OR′ substituiert sein kann, worin R′ einen Alkyl- oder Alkylcarboxy-Rest mit 1 bis 3 C-Atomen bedeutet. Ebenso bevorzugt sind Verbindungen, in denen $R_2$ Wasserstoff und $R_1$ einen Silylrest bedeutet, der mit Alkylgruppen, die 1 bis 4 C-Atome enthalten, mit Alkoxygruppen, die 1 bis 5 C-Atome enthalten oder mit Chlor substituiert ist.

Nach Beendigung der Reaktion wird die Mischung abgekühlt und, sofern sie flüssig bleibt, durch Abfiltrieren, Abzentrifugieren oder andere geeignete Methoden von festen Partikeln getrennt. Das Filtrat wird nun, gegebenenfalls unter Anwendung von Unterdruck, fraktioniert destilliert. Sofern die Hauptfraktion kein ausreichend reines Produkt enthält, wird diese erneut fraktioniert destilliert. Wenn die Reaktionsmischung beim Abkühlen erstarrt, wird sie zweckmässig mit geeigneten leicht-flüchtigen Lösungsmitteln extrahiert, entweder durch mehrfaches Digerieren oder beispielsweise in einem Soxhlet-Apparat. Geeignete Lösungsmittel sind beispielsweise Dichlormethan, Chloroform, Trichlorethylen, Tetrachlorkohlenstoff, niedrig siedende, jedoch bei Zimmertemperatur flüssige fluorierte Kohlenwasserstoffe, die auch noch Chlormoleküle enthalten können, Ether, z. B. Diethylether, Tetrahydrofuran, Glykoldimethylether. Aus der Extraktion wird nun zunächst das Lösungsmittel teilweise oder vollständig abgedampft und das Produkt durch Umkristallisation gereinigt oder das Lösungsmittel vollständig abgedampft und das Produkt durch fraktionierte Destillation, wie oben beschrieben, in reiner Form gewonnen. Das im Filter- bzw. Zentrifugen- oder Extraktions-Rückstand vorhandene Metall bzw. die metallhaltigen Partikel werden gegebenenfalls nach Reinigung, beispielsweise Waschen mit geeigneten Lösungsmitteln, als Katalysator wieder verwendet.

Mit dem erfindungsgemässen Verfahren werden mit guter Selektivität die einfachen Additionsverbindungen des fluorierten Alkyliodids mit dem entsprechenden Alken erhalten, die folgender Formel entsprechen:

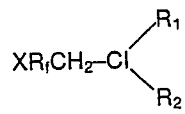

worin $R_f$, $R_1$, $R_2$ und X die weiter oben genannte Bedeutung haben. Nur in untergeordnetem Masse können Reaktionsprodukte auftreten, bei denen das fluorierte Alkyliodid mit zwei Alkenen reagiert hat, wobei zum Beispiel folgende Verbindungen entstehen können:

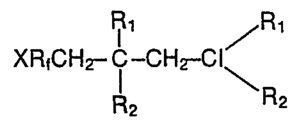

Wie weiter oben bereits ausgeführt, kann jedoch die Bildung von Verbindungen der zuletzt genannten Formel durch Anwendung eines Überschusses des fluorierten Alkyliodids zurückgehalten werden.

Die nach dem neuen Verfahren erzeugten Verbindungen sind wertvolle Zwischenprodukte, sie können beispielsweise verarbeitet werden, indem nach bekanntem Verfahren, wie zum Beispiel in der DE-OS 2 834 795 beschrieben, das Iodatom im Molekül in eine Hydroxylgruppe übergeführt wird. Ungesättigte Verbindungen werden erhalten durch Abspalten von Iodwasserstoff mit Alkalien, wie beispielsweise Natrium oder Kaliumhydroxid, Natrium- oder Kaliumcarbonat, wobei man zweckmässig in wässrig-alkoholischer oder rein alkoholischer Lösung, beispielsweise in Methanol oder Ethanol, arbeitet, wobei die Reaktion im Falle von wasserhaltigen Medien durch Einsatz von Phasentransfer-Katalysatoren wie Tetraalkylammonium-Salze oder Tetraalkylphosphonium-Salze noch verbessert wird. Verbindungen mit Doppelbindungen jeweils am Molekülende sind als Comonomere für die Polymerisation von fluorhaltigen ungesättigten Kohlenwasserstoffen, beispielsweise Tetrafluorethylen, geeignet. Andere aus den erfindungsgemäss hergestellten Verbindungen erhaltene Stoffe dienen zur Herstellung von Textilveredlungsmitteln, beispielsweise zur Oleophobierung oder Hydrophobierung, als Feuerlöschmittel oder als besonders beständige Emulgatoren, beispielsweise für die Elektrolyse.

Nachfolgende Beispiele sollen die Erfindung näher erläutern.

Vergleichsversuch A und Beispiele 1 bis 11

Es wird ein Schüttelautoklav aus $V_2A$-Edelstahl verwendet, der mit Polytetrafluorethylen ausge-

kleidet ist und 250 $cm^3$ Inhalt besitzt. In diesen Autoklaven werden die in nachstehender Tabelle angegebenen Verbindungen der Formel $XR_fI$, Metalle in Pulverform sowie gegebenenfalls Wasser in den aus der Tabelle ersichtlichen Mengen gegeben. Der Autoklav wird nun verschlossen, zunächst mit Stickstoff, dann mit Ethylen gespült und schliesslich ein Ethylendruck von 2 MPa im Autoklav eingestellt. Nun wird der Autoklav unter Schütteln aufgeheizt bis zu der in der Tabelle angegebenen Temperatur und während der ebenfalls dort verzeichneten Dauer auf dieser Temperatur gehalten, wobei auch der Ethylendruck konstant gehalten wird. Das durch die Reaktion verbrauchte Ethylen wird also ständig nachgeliefert, so dass im Autoklav stets ein molarer Überschuss an Ethylen im Verhältnis zur eingesetzten Verbindung $XR_fI$ vorhanden ist. Die Metalle Ruthenium und Platin werden in fein verteilter Form auf Aktivkohle niedergeschlagen verwendet. Die Aktivkohle enthält etwa 5 Gew.-% Ruthenium bzw. Platin. Die angewendete Menge wird so bemessen, dass 2 bzw. 1 Mol-%, bezogen auf jene der beiden miteinander reagierenden Verbindungen, die mit geringerer Molzahl eingesetzt wird, von dem Metall zur Anwendung kommt.

Nach Beendigung der Reaktion wird der Autoklav abgekühlt, entspannt und geöffnet. Der Autoklaveninhalt wird nun, gegebenenfalls nach Aufschmelzen, filtriert. Von einer Probe des Filtrates wird ein $^{19}F$-Kernresonanzspektrum aufgenommen und anhand dieses Spektrums der prozentuale Umsatz, bezogen auf die eingesetzten $-CF_2I$-Gruppen, ermittelt. Eine weitere Probe des Filtrates wird beim Versuch A und bei den Beispielen 1 bis 8 gaschromatographisch untersucht. Die gefundenen Flächenprozente im Gaschromatogramm, die angenähert den tatsächlich gebildeten Molprozenten entsprechen, sind in nachfolgender Tabelle angegeben. Hierbei bedeuten:

$m_1$ = Reaktionsprodukt von einem Molekül $XR_fI$ mit einem Molekül der Formel

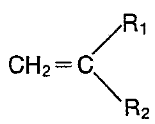

wobei eine Verbindung folgenden Typs entsteht:

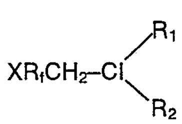

$m_2$ = Reaktionsprodukt von einem Molkül $XR_fI$ mit zwei Molekülen einer Verbindung der Formel

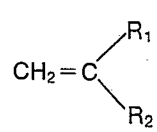

wobei im allgemeinen eine Verbindung folgender Formel entsteht:

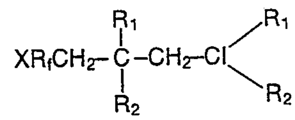.

$XR_fI$ bedeutet eine Verbindung, in der das Iodatom der Verbindung $XR_fI$ durch Wasserstoff ersetzt ist.

Beim Beispiel 9 wird nicht abfiltriert sondern das katalysatorhaltige Rohprodukt in einer Soxhlet-Apparatur mit siedendem Dichlormethan extrahiert. Nach Abdampfen des Lösungsmittels und Trocknung wird ein farbloses kristallines Produkt erhalten, welches gaschromatographisch untersucht wird und zu 97,8% aus einer Verbindung folgender Formel besteht:

$$ICH_2CH_2(CF_2)_4CH_2CH_2I$$

In diesem Falle wurde eine Verbindung $XR_fI$ eingesetzt, in der X ein zweites Iodatom bedeutet. Es ist also bei der Reaktion mit Ethylen die Verbindung entstanden, bei der je ein Molekül Ethylen mit einer $-CF_2I$-Gruppe reagiert hat. Sinngemäss wurde daher der gaschromatographisch ermittelte Wert in nachfolgender Tabelle in der Spalte unter $m_1$ eingetragen

Beispiel 12

Es wird ein heizbarer Schüttelautoklav aus $V_2A$-Stahl, welcher 4000 $cm^3$ Inhalt hat, verwendet. Nach Einfüllung aller in nachfolgender Tabelle angegebener Stoffe in den dort verzeichneten Mengen wird der Autoklav verschlossen, mit Stickstoff gespült, die Gaszufuhr unterbrochen und unter Schütteln auf 180 °C während 11 h erwärmt. Hierbei entsteht im Autoklav ein autogener Druck von 1,5 MPa. Nach Beendigung der Reaktionsdauer wird abgekühlt, entspannt, der Autoklaveninhalt filtriert und das Filtrat einer fraktionierten Destillation unterworfen. Bei einem Druck von 6,7 kPa geht bei einer Temperatur von 95 bis 97 °C das Reaktionsgemisch von einem Molekül Perfluorbutyliodid mit einem Molekül 1,1,2-Trihydro-1-perfluorhexen, welches folgender Formel entspricht:

$$C_4F_9CHI–CH_2C_4F_9$$

über. Beim gleichen Druck geht die nächste Fraktion bei 110 bis 120 °C über und enthält das Reaktionsprodukt von einem Molekül Perfluoralkyliodid mit 2 Molekülen 1,1,2-Trihydro-1-perfluorhexen, welches folgender Formel entspricht

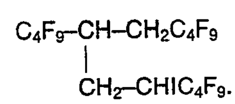

Beide Fraktionen werden gewogen, anhand des Gewichtes wird die Ausbeute in Prozenten der Menge berechnet, die theoretisch bei vollständiger Umsetzung aus dem im molaren Unterschuss vorhandenen 1,1,2-Trihydro-1-perfluorhexen hätte entstehen können. Der Wert für das Umsetzungs-

Tabelle

| Vergleichs-versuch-Beispiel Nr. | $XR_fI$ | mol | $CH_2=C<^{R_1}_{R_2}$ | mol | Metall[2+)] Mol-% | Wasser[2+)] Mol-% | Temp. °C | Druck MPa | Dauer h | Umsatz[3+)] % | Produkteigenschaften[4+)] (Flächen-%) $m_1$ | $m_2$ | $XR_fI$ | $XR_fH$ |
|---|---|---|---|---|---|---|---|---|---|---|---|---|---|---|
| A | I | 0,2 | a | >0,2 | – | – | 190 | 2 | 11 | 91 | 83,8 | 1,2 | 9,0 | 5,5 |
| 1 | I | 0,2 | a | >0,2 | Zn 2 | – | 130 | 2 | 8,5 | 100 | 96,1 | 3,5 | – | 0,3 |
| 2 | I | 0,2 | a | >0,2 | Cr 2 | – | 145 | 2 | 6 | 92,1 | 90,5 | 1,3 | 7,9 | 0,3 |
| 3 | I | 0,2 | a | >0,2 | Ni 2 | – | 125 | 2 | 12 | 98,6 | 97,5 | 1,2 | 0,9 | 0,5 |
| 4 | I | 0,2 | a | >0,2 | Ag 2 | – | 125 | 2 | 11 | 95,5 | 93,8 | 1,3 | 4,0 | 0,8 |
| 5 | I | 0,2 | a | >0,2 | Re 2 | – | 135 | 2 | 12 | 92,3 | 90,5 | 1,8 | 7,1 | 0,4 |
| 6 | I | 0,2 | a | >0,2 | Ru[+)] 2 | – | 100 | 2 | 11 | 84,8 | 83,7 | 0,2 | 15,2 | 0,9 |
| 7 | I | 0,2 | a | >0,2 | Pt[+)] 2 | – | 90 | 2 | 4 | 78,7 | 78,4 | – | 21,2 | 0,2 |
| 8 | II | 0,4 | a | >0,4 | Ru[+)] 2 | 5 | 120 | 2 | 6 | 100 | 99,4 | n.b. | n.b. | n.b. |
| 9 | III | 0,68 | a | >0,68 | Ru[+)] 1 | – | 170 | 2 | 33 | 98,5 | 97,8 | n.b. | n.b. | n.b. |
| 10 | II | 0,3 | c | 0,1 | Pt[+)] 1 | – | 155 | n.b. | 12 | 23,9 | 22,8 | 4,9 | 72,3 | n.b. |
| 11 | II | 0,2 | d | 0,1 | Ru[+)] 2 | 20 | 150 | n.b. | 12 | 21,2 | 36,3 | 2,8 | 60,3 | n.b. |
| 12 | II | 6 | b | 1 | Ru[+)] 2 | 5 | 180 | 1,5 | 11 | 35 | 79,9[5+)] | 8,3[5+)] | n.b. | n.b. |
| 13 | IV | 0,1 | e | 0,15 | Ni 2 | 10 | 122–170 | 0,1 | 7 | 98 | 92,6 | 5,1[6+)] | 2,3 | n.b. |
| 14 | V | 0,2 | f | 0,2 | Cu 5 | 50 | 85–100 | 0,1 | 5,5 | 69,7 | 68,1 | 2,4 | 29,5 | n.b. |
| B | IV | 0,1 | e | 0,1 | Cu 5 | [7+)] | 110 | 0,1 | 5 | 41,3 | 28,3 | 11,3[6+)] | 57,9 | 2,5 |
| C | IV | >0,1 | a | 0,1 | Cu 66[8+)] | [7+)] | 110 | 0,1 | 11 | 98 | 57,0 | 40,8[9+)] | 1,9 | n.b. |

+) auf Aktivkohle
2+) bezogen auf jene der beiden miteinander reagierenden Verbindungen, die mit geringerer Molzahl eingesetzt wird
3+) bezogen auf die in der Verbindung $XR_fI$ vorhandenen $-CF_2I$-Gruppen
4+) gaschromatographisch ermittelt
5+) Ausbeute in % der theoretischen Menge, bezogen auf eingesetzte Verbindung b
6+) kein Doppel-Additionsprodukt, sondern eine Mischung von $C_8F_{17}CH_2$–CH–CH–$C_5H_{11}$ und $C_8F_{17}CH_2CH_2CH_2C_5H_{11}$
7+) kein Wasser verwendet, sondern 50 $cm^3$ Dimethylsulfoxid
8+) dies entspricht ⅓ der stöchiometrisch zur Bildung von $R_fCu + CuI$ notwendigen Menge
9+) verschiedene Nebenprodukte. Eine Anlagerungsverbindung von 2 Molekülen Ethylen an $C_8F_{17}I$ ist nicht nachweisbar
n.b. nicht bestimmt.

produkt von je einem Molekül Perfluorbutyliodid und 1,1,2-Trihydro-1-perfluorhexen ist in nachfolgender Tabelle unter $m_1$, der Wert für das Umsetzungsprodukt mit 2 Molekülen 1,1,2-Trihydro-1-perfluorhexen ist in nachfolgender Tabelle unter $m_2$ aufgeführt.

Beispiele 13 und 14 sowie Vergleichsversuche B und C

Es wird ein temperierbarer Glaskolben von 250 $cm^3$ Inhalt verwendet, der mit Rührer, Rückflusskühler, Thermometer und einem Gaseinleitungsrohr versehen ist. In den Glaskolben werden die in nachfolgender Tabelle aufgeführten Reaktionspartner und der Katalysator in den verzeichneten Mengen gegeben, durch das Gaseinleitungsrohr, bei den Beispielen 13 und 14 sowie beim Vergleichsversuch B Argon, beim Vergleichsversuch C Ethylen, eingeleitet und der Kolbeninhalt unter Rühren auf die in der Tabelle angegebene Temperatur erwärmt und während der ebenfalls ersichtlichen Zeit auf dieser Temperatur gehalten. Bei den Beispielen 13 und 14 wird am Rückfluss gekocht, wobei sich die Temperatur der Reaktionsmischung im Laufe der Zeit erhöht. Am Ende der Reaktion wird bei den Beispielen 13 und 14 die Mischung etwas abgekühlt und filtriert. Von Proben des Filtrats wird das $^{19}F$-Kernresonanzspektrum aufgenommen und die Zusammensetzung gaschromatographisch bestimmt. Aufgrund der ermittelten Werte beträgt die Selektivität der Reaktion bezüglich der Bildung des Mono-Additionsproduktes: beim Beispiel 13: 94,8%, beim Beispiel 14: 96,6%. In beiden Fällen wird die Hauptmenge des Filtrats der Kurzweg-Destillation im Vakuum unterworfen. Es wurden gefunden:

Beispiel 13: Die Hauptfraktion geht bei 116 °C und 133 Pa über, sie besteht aus reinem $C_8F_{17}CH_2CHIC_6H_{13}$; Ausbeute 85,5% der theoretischen Menge, bezogen auf umgesetztes $C_8F_{17}I$.

Beispiel 14: Die Hauptfraktion geht bei 130 °C und 1,87 kPa über, sie besteht aus reinem $C_6F_{13}CH_2CHICH_2OCOCH_3$, Ausbeute 75,9% der theoretischen Menge, bezogen auf umgesetztes $C_6F_{13}I$.

Bei den Vergleichsversuchen B und C wird nach Ablauf der Reaktionszeit gekühlt und vom Rohprodukt das $^{19}F$-Kernresonanzspektrum ermittelt. Die Reaktionsmischung wird nun mit 30 $cm^3$ Diethylether und 100 $cm^3$ Wasser gerührt, filtriert, die etherhaltige Phase abgetrennt und mit Wasser ausgeschüttelt, um restliches Dimethylsulfoxid vollständig zu entfernen und gaschromatographisch untersucht. Die gefundenen Werte sind aus nachfolgender Tabelle ersichtlich. Die Selektivität der Reaktion bezüglich der Bildung des Mono-Additionsproduktes beträgt:

beim Vergleichsversuch B: 67,2%
beim Vergleichsversuch C: 58,3%.

In der Tabelle bedeuten:

I = Gemisch verschiedener Perfluoralkyliodide mit folgender Kettenlängen-Verteilung: 37,5 Gew.-% $C_6$; 37,9 Gew.-% $C_8$; 17,5 Gew.-% $C_{10}$; 6,3 Gew.-% $C_{12}$;
II = 1-Iodperfluorbutan;
III = 1,4-Diiodperfluorbutan;
IV = 1-Iodperfluoroctan;
V = 1-Iodperfluorhexan;
a = Ethylen $CH_2=CH_2$;
b = 1,1,2-Trihydro-1-perfluorhexen $CH_2=CH-C_4F_9$;
c = Chlorethen (Vinylidenfluorid)
d = 1,1-Difluorethen (Vinylidenfluorid)
e = 1-Octen
f = 2-Propen-1-ol-acetat (Essigsäureallylester).

**Patentansprüche**

1. Verfahren zur Herstellung von fluoralkylsubstituierten Iod-Alkanen durch Reaktion einer Verbindung der Formel

$$XR_fI \qquad (I),$$

worin bedeuten:

$R_f$ einen perfluorierten Alkylenrest, geradkettig mit 1 bis 15 C-Atomen, verzweigt mit 3 bis 15 C-Atomen oder cyclisch mit 4 bis 8 C-Atomen,

X H; F; Cl; Br oder I,

mit einer Verbindung der Formel

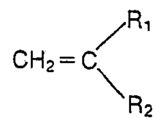

worin $R_1$ und $R_2$ gleich oder verschieden sein können und jedes für sich bedeutet: Wasserstoff, Fluor, Chlor, einen Perfluoralkylrest mit 1 bis 12 C-Atomen, in dem ein Fluoratom durch Wasserstoff oder Chlor ersetzt sein kann; einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann, mit F; Cl; –OH oder –OR′, worin R′ einen Alkyl- oder Alkylcarboxyrest mit 1 bis 5 C-Atomen bedeutet, in der Wärme, bei Normaldruck oder Überdruck, in Gegenwart eines Katalysators, dadurch gekennzeichnet, dass die Reaktion bei 80 bis 180 °C durchgeführt wird, wobei als Katalysator mindestens ein Metall, das im Periodensystem der Elemente eine der Ordnungszahlen 24 bis 30; 42 bis 48 oder 74 bis 79 aufweist, in fein verteilter Form eingesetzt wird.

2. Verfahren nach Anspruch 1, dadurch gekennzeichnet, dass als Katalysator Chrom, Mangan, Nickel, Ruthenium, Rhodium, Palladium oder Platin verwendet wird.

3. Verfahren nach Anspruch 1 oder 2, dadurch gekennzeichnet, dass sich das Katalysator-Metall auf einem feinteiligen, inerten Trägermaterial befindet.

4. Verfahren nach einem oder mehreren der Ansprüche von 1 bis 3, dadurch gekennzeichnet, dass bei einer Temperatur von 100 bis 150 °C gearbeitet wird.

5. Verfahren nach einem oder mehreren der Ansprüche von 1 bis 4, wobei eine der beiden reagierenden Verbindungen, entweder (I) oder (II), in geringerer Molzahl eingesetzt wird als die anderen reagierenden Verbindungen, dadurch gekennzeichnet, dass 0,1 bis 10 Mol-%, bezogen auf jene der beiden reagierenden Verbindungen, die mit geringerer Molzahl eingesetzt wird, von dem als Katalysator verwendeten Metall angewendet werden.

6. Verfahren nach einem oder mehreren der Ansprüche von 1 bis 5, wobei eine der beiden reagierenden Verbindungen, entweder (I) oder (II), in geringerer Molzahl eingesetzt wird als die andere reagierende Verbindung, dadurch gekennzeichnet, dass 1 bis 100 Mol-%, bezogen auf jene der beiden reagierenden Verbindungen, die mit geringerer Molzahl eingesetzt wird, Wasser der Reaktionsmischung zugesetzt werden.

**Claims**

1. A process for preparing fluoroalkyl-substituted iodalkanes by reacting a compound of the following formula:

$$XR_fI \qquad (I)$$

in which

$R_f$ denotes a perfluorinated alkylene radical which is straight-chain and has 1 to 15 carbon atoms, which is branched and has 3 to 15 carbon atoms or which is cyclic and has 4 to 8 carbon atoms, and

X denotes H; Cl; Br or I,

with a compound of the following formula

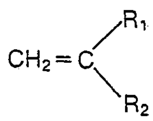

in which $R_1$ and $R_2$ can be identical or different and each denotes:

hydrogen, fluorine, chlorine, a perfluoroalkyl radical which has 1 to 12 carbon atoms and in which a fluorine atom can be replaced by hydrogen or chlorine, an alkyl radical having 1 to 20 carbon atoms, an alkenyl radical having 2 to 20 carbon atoms, an aryl radical having 6 to 10 carbon atoms or an arylalkyl radical having 7 to 12 carbon atoms, and a substituent of the last four types can in turn be substituted by F, Cl, –OH or –OR′, in which R′ denotes an alkyl or alkylcarboxyl radical having 1 to 5 carbon atoms, under heat and under atmospheric pressure or superatmospheric pressure in the presence of a catalyst, characterised by carrying out the process at 80 to 180 °C and using as said catalyst at least one metal which, in the periodic table of the elements, has one of the atomic numbers 24 to 30, 42 to 48 or 74 to 79 and is in finely divided form.

2. The process as claimed in claim 1, characterised in that the catalyst is chromium, manganese, nickel, ruthenium, rhodium, palladium or platinum.

3. The process as claimed in claim 1 or 2, characterised in that the catalyst metal is present on a finely divided, inert support material.

4. The process as claimed in one or more of claims 1 to 3, characterised in that the reaction is carried out at a temperature of 100 to 150 °C.

5. The process as claimed in one or more of claims 1 to 4 and in which one of the two reacting compounds, either (I) or (II) is used in a smaller number of moles than the other reacting compound, characterised in that the metal used as the catalyst is used in an amount of 0.1 to 10 mol%, based on whichever of the two reacting compounds is used in the smaller number of moles.

6. The process as claimed in one or more of claims 1 to 5 in which one of the two reacting compounds, either (I) or (II) is used in a smaller number of moles than the other reacting compound, characterised in that water is added to the reaction mixture in an amount of 1 to 100 mol%, based on whichever of the two reacting compounds is used in the smaller number of moles.

**Revendications**

1. Procédé pour préparer des iodo-alcanes à substituant(s) fluoro-alkyles, par réaction d'un composé de formule

$$XR_fI \qquad (I)$$

dans laquelle

$R_f$ désigne un reste alkylène perfluoré, linéaire comportant 1 à 15 atomes de carbone, ramifié comportant 3 à 15 atomes de carbone ou bien cyclique comportant 4 à 8 atomes de carbone,

X représente un atome de H, F, Cl, Br ou I,

avec un composé de formule

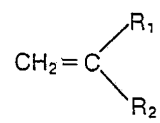

dans laquelle $R_1$ et $R_2$ peuvent être identiques ou différents et représentent chacun:

un atome d'hydrogène, de fluor ou de chlore, un reste perfluoro-alkyle ayant 1 à 12 atomes de carbone, dans lequel un atome de fluor peut être remplacé par un atome d'hydrogène ou de chlore; un reste alkyle ayant 1 à 20 atomes de carbone, un reste alcényle ayant 2 à 20 atomes de carbone, un reste aryle ayant 6 à 10 atomes de carbone ou un reste arylalkyle ayant 7 à 12 atomes de carbone, un substituant des quatre catégoieres citées en dernier lieu pouvant être pour sa part substitué par F, Cl, –OH ou –OR′, où R′ représente un reste alkyle ou alkylcarboxy ayant 1 à 5 atomes de carbone, à chaud, sous la pression normale ou sous une pression supérieure à la pression normale, en présence d'un catalyseur, procédé caractérisé en ce qu'on conduit la réaction entre 80 et 180 °C, en utilisant, sous forme finement divisée, comme catalyseur au moins un métal qui présente, dans le système ou tableau périodique

des éléments, un numéro atomique allant de 24 à 30, de 42 à 48 ou de 74 à 79.

2. Procédé selon la revendication 1, caractérisé en ce qu'on utilise comme catalyseur du chrome, du manganèse, du nickel, du ruthénium, du rhodium, du palladium ou du platine.

3. Procédé selon la revendication 1 ou 2, caractérisé en ce que le métal constituant le catalyseur se trouve sur une matière inerte de support en fines particules.

4. Procédé selon une ou plusieurs des revendications 1 à 3, caractérisé en ce qu'on travaille à une température de 100 à 150 °C.

5. Procédé selon une ou plusieurs des revendications 1 à 4, dans lequel on utilise l'un des deux composés destinés à réagir, soit (I), soit (II), en une quantité molaire inférieure à celle de l'autre composé destiné à réagir, procédé caractérisé en ce qu'on utilise 0,1 à 10 moles % du métal servant de catalyseur, sur la base de celui des deux composés destinés à réagir que l'on utilise en la plus faible quantité molaire.

6. Procédé selon une ou plusieurs des revendications 1 à 5, selon lequel on utilise l'un des deux composés mis à réagir, soit (I), soit (II), en une quantité molaire plus faible que celle de l'autre composé destiné à réagir, procédé caractérisé en ce qu'on ajoute au mélange réactionnel 1 à 100 moles % d'eau, sur la base de celui des deux composés destinés à réagir que l'on utilise en la plus faible quantité molaire.